# EUROPEAN PATENT APPLICATION

(11) **EP 4 509 518 A1**
(43) Date of publication of application: **19.02.2025**
(21) Application number: 23786464.0
(22) Date of filing: 11.04.2023
(51) Int. Cl.: C07K 14/015, C12N 15/35, C12N 15/864, C12N 5/10, C12N 7/01, A61K 38/16, A61K 48/00, A61P 27/02, A61P 27/16

(54) **FUSION TYPE ADENO-ASSOCIATED VIRUS AND USE THEREOF**

(30) Priority: 12.04.2022 CN 202210383577
(71) Applicant: Starrygene Therapeutics Co., Ltd., Hefei, Anhui 230031 (CN)
(72) Inventor: CAI, Yuan, Hefei, Anhui 230031 (CN); ZHU, Ying, Hefei, Anhui 230031 (CN); ZHANG, Jie, Hefei, Anhui 230031 (CN); ZHONG, Guisheng, Shanghai 201210 (CN); CHU, Cenfeng, Shanghai 201210 (CN)
(74) Representative: J A Kemp LLP
(86) International application number: PCT/CN2023/087598
(87) International publication number: WO 2023/198050

(57) **Abstract**

The present invention relates to the field of biological medicines. Disclosed are a fusion adeno-associated virus and a use thereof in otological disease and ophthalmic diseases. The fusion adeno-associated virus comprises a fusion peptide fragment formed by fusion of peptide fragments of serotypes AAV1, AAV2, AAV6 and AAV7, or a variant thereof, and can efficiently infect an RPE layer for the treatment of ophthalmic diseases. The fusion adeno-associated virus has a wide application prospect in the treatment of ophthalmic diseases as a safe vector.

## Description

### TECHNICAL FIELD

The present invention relates to the field of biomedicine, and relates to a fusion adeno-associated virus and use thereof, particularly in the prevention and/or treatment of otological disease and/or ophthalmic diseases.

### BACKGROUND

There are about 7,000 known human diseases to date, among which approximately 80% are caused by genetic issues, and more than 300 million people in the world are deeply troubled by genetic diseases. Current chemotherapy, protein therapy and the like can only treat less than 500 types of human diseases, and the therapy against diseases caused by genetic issues are usually incompetent. Therefore, it is necessary to develop new drugs and treatment schemes.

Gene therapy is an emerging treatment method which could delivers functional genes into a patient's body to counteract or replace dysfunctional genes, thereby curing diseases without chemical intervention, radiation therapy, or surgery. Gene therapy introduces genetic material into target cells through viral or non-viral vectors to treat or prevent diseases by correcting or supplementing defective genes, and the effects of treatment can be long-lasting without repeated intervention. Gene therapy can be achieved by *ex vivo* or *in vivo* strategies, in which *ex vivo* gene therapy is to collect target cells from a patient, genetically modify and return to the patient; *in vivo* gene therapy is to deliver genetic material directly into the patient's target organ or tissue. Various types of gene delivery strategies have been used to treat various diseases such as cancer, blindness, immune diseases and neuronal diseases at present.

Viral vectors are the most commonly used gene therapy delivery vectors because of their high efficiency in entering cells and introducing genetic material, and commonly used are adenoviruses, retroviruses, lentiviruses, and adeno-associated viruses (AAV) and the like. Nowadays, AAV vectors have achieved excellent results in clinical trials of gene therapy *in vivo,* and retroviral and lentiviral vectors are the preferred vectors in clinical trials of gene therapy *in vitro.* The primary factor that AAV can successfully serve as a therapeutic gene delivery vector is its low immunogenicity and lack of pathogenicity. Multiple commercial drug approvals and ongoing clinical trials demonstrate that AAV vectors have become one of the major gene delivery tools for gene therapy. In 2012, Glybera was the first AAV therapy approved in Europe for the treatments of lipoprotein lipase deficiency. The market availability of these drugs clearly demonstrates the safety and effectiveness of AAV-based treatment methods. Thereafter, Luxturna for the treatment of Leber congenital amaurosis and Zolgensa for the treatment of spinal muscular atrophy (SMA) were approved by the U. S. Food and Drug Administration (FDA) in 2017 and 2019, respectively. Current clinical researches focus on the treatment of single gene diseases through gene substitution, gene silencing or gene editing. In addition to being used in the treatment of various diseases such as cancer, blindness, immune and neuronal diseases, AAV is also expected to be used in the development of vaccines.

Although AAV is characterized by low immunogenicity and low pathogenicity, and AAV-based gene therapy has also achieved surprising success, up to 50% patients are currently excluded from this treatment method due to the preexisting neutralizing antibodies of natural AAV capsid protein in human immune system. To circumvent such an immune disorder, methods of evading preexisting neutralizing antibodies with engineered AAV capsids or temporarily removing neutralizing antibodies prior to treatment can be adopted. The engineered AAV capsid can not only evade neutralizing antibodies, but also target the AAV to the expected tissue cell type, thereby achieving the purpose of accurate treatment. Directed evolution is a powerful tool for current modification of AAV capsid, and in principle, directed evolution uses a high-throughput technical means to introduce mutations into genes and encoded proteins thereof, and simulates natural evolution to greatly accelerate protein diversification and selection processes. The method comprises three basic steps: establishing a sequence diversity library of a target gene, screening from a protein library encoded by a mutant gene library, and amplifying a gene sequence encoding a required character. These three steps constitute one round of directed evolution, and multiple rounds of evolution screening can be performed iteratively until a specific gene/protein with improved characteristics is obtained.

However, the current use of adeno-associated virus as a vector for gene therapy in auditory systems still has the disadvantages of poor tissue specificity, low infection efficiency and the like, for example, it is known that adeno-associated virus vectors also infect supporting cells while infecting hair cells; supporting cells and hair cells respectively express different pathogenic genes, and the non-specificity of the vector infection has an adverse effect on disease therapy. Therefore, to achieve accurate gene therapy, an efficient and specific novel recombinant adeno-associated virus vector is required.

### SUMMARY

The present invention aims to overcome the defects of the prior art, and provides an emerging novel adeno-associated virus vector capable of efficiently and specifically delivering genes in cochlea, eye retina and other tissues for gene therapy. The present invention infects mouse cochlea *in vivo* with the modified novel adeno-associated virus vector, and the target gene can be mediated to be specifically expressed in cochlear supporting cells, thereby specifically labeling or controlling the supporting cells. The novel adeno-associated virus has wide range of application value and market prospect in the aspects of structure and function analysis of cochlear supporting cells, establishment of disease models, gene therapy and the like. Meanwhile, after the virus was injected into the mouse eyeball through a vitreous body, it was found that the viral vector has more excellent infection characteristics compared with existing vectors, and also has wide application value and market prospect in the aspects of structure and function analysis of ophthalmic cells, establishment of disease models, gene therapy and the like.

One object of the present invention is to provide a fusion adeno-associated virus capsid protein comprising a fusion peptide fragment of peptide fragments of serotypes AAV1, AAV2, AAV6 and AAV7, or a variant thereof.

Another object of the present invention is to provide a nucleic acid encoding the fusion adeno-associated virus capsid protein as described above.

Another object of the present invention is to provide a construct comprising the nucleic acid as described above.

Another object of the present invention is to provide a host cell comprising a construct or the genome integrates an exogenous nucleic acid as described above, or comprising a fusion adeno-associated virus as described above.

Another object of the present invention is to provide a fusion adeno-associated virus, wherein the capsid structure of the fusion adeno-associated virus comprises the fused adeno-associated virus capsid protein described in any one of the above.

A host cell transformed using the fusion adeno-associated virus as described above.

Another object of the present invention is to provide a fusion adeno-associated virus vector system comprising a packaging plasmid comprising a nucleic acid or a nucleic acid fragment as described above.

Another object of the present invention is to provide a fusion adeno-associated virus obtained by virus packaging of the fusion adeno-associated virus vector system as described above.

Another object of the present invention is to provide a pharmaceutical composition comprising the fusion adeno-associated virus as described above and a pharmaceutically acceptable auxiliary material.

Another object of the present invention is to provide use of the fusion adeno-associated virus capsid protein, nucleic acid, construct, fusion adeno-associated virus, host cell, fusion adeno-associated virus vector system, pharmaceutical composition or conjugate as described above in the preparation of a medicament for treating diseases; preferably, in the preparation of a medicament for gene therapy of diseases.

Compared with the prior art, the present invention has the following advantages and beneficial effects: (1) provided is a supporting cell specific novel adeno-associated virus vector, which can mediate the specific expression of a target gene in cochlear supporting cells of newborn mice; (2) provided is a novel adeno-associated virus vector, which can mediate the specific expression of the target gene in hair cells in cochlea of adult mice; (3) provided is a novel adeno-associated virus vector, which can mediate the specific expression of the target gene in a retinal RPE layer of adult mice; (4) the novel adeno-associated virus vector mediates the expression of the target gene more flexibly, safely, conveniently and cost-effectively than a traditional transgenic method; and (5) the novel specific adeno-associated virus vector makes it possible to develop gene therapy for accurately treating otological and ophthalmologic related diseases.

### BRIEF DESCRIPTION OF THE DRAWINGS

FIG. 1 shows an *in vivo* screening procedure of a DNA family shuffling library.
FIG. 2 shows a computer simulated AAV-M9 structure diagram.

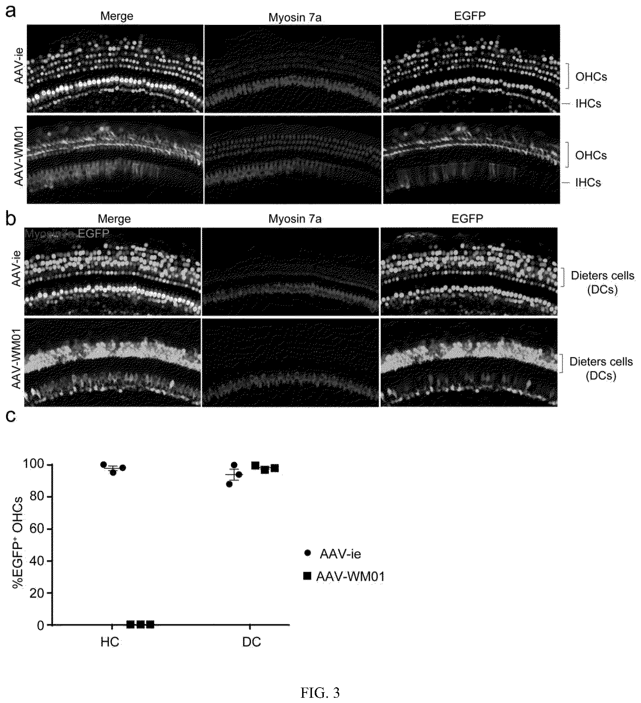

FIG. 3 shows the infection characteristics of adeno-associated viruses in neonatal mice. FIG. 3a shows immunostaining photographs of green fluorescent EGFP and hair cell specific marker protein Myo7a after removal and dissection of cochlear tissues after 14 days after injection of AAV-ie and AAV-M9 into cochlea in neonatal mice. The pictures of the hair cell layer are selected. FIG. 3b shows immunostaining photographs of green fluorescent EGFP and hair cell specific marker protein Myo7a after removal and dissection of cochlear tissue after 14 days after injection of AAV-ie and AAV-M9 into cochlea in neonatal mice. The pictures of the supporting cell layer are selected. FIG. 3c shows data statistics on hair cells and Dieters cells in FIGs. 3a, 3b.
FIG. 4 shows the safety of adeno-associated viruses in neonatal mice. FIG. 4a shows immunostaining photographs of hair cell specific marker protein Myo7a after removal and dissection of the cochlear tissue after 14 days after injection of AAV-M9 into cochlea in neonatal mice and the cochlear tissue of normal mice. FIG. 4b shows data statistics on the outer hair cells and inner hair cells in FIG. 4a.
FIG. 5 shows the infection characteristics of adeno-associated viruses in adult mice. FIG. 5a shows immunostaining photographs of green fluorescent EGFP and hair cell specific marker protein Myo7a after removal and dissection of cochlear tissues after 14 days after injection of AAV-ie and AAV-M9 into cochlea in adult mice. FIG. 5b shows data statistics on the outer hair cells in FIG. 5a. FIG. 5c shows the data statistics on the inner hair cells in FIG. 5a.
FIG. 6 shows the safety of adeno-associated viruses in adult mice. FIG. 6a shows immunostaining photographs of hair cell specific marker protein Myo7a after removal and dissection of the cochlear tissue after 14 days after injection of AAV-M9 into cochlea in adult mice and the cochlear tissue of normal mice. FIG. 6b shows data statistics on the outer hair cells and inner hair cells in FIG. 6a.
FIG. 7 shows the infection of AAV-M9-CMV-EGFP in the RPE layer of ocular.
FIG. 8 shows characteristics of AAV-M9-CMV-EGFP for ocular transfection in non-human primates.

### DETAILED DESCRIPTION

The following describes the embodiments of the present invention through specific examples, and other advantages and effects of the present invention will be readily apparent to the skilled in the art from the disclosure of the present specification. The present invention may also be implemented or applied through other different specific embodiments, and various details in this specification can also be modified or changed in various ways based on different perspectives and applications without departing from the spirit of the present invention.

The invention provides a fusion adeno-associated virus capsid protein comprising a peptide fragment (fusion peptide fragment or chimeric peptide fragment) formed by fusion of peptide fragments of serotypes AAV1, AAV2, AAV6 and AAV7, or a variant thereof.

In some preferred embodiments, in the fusion adeno-associated virus capsid protein, the fusion peptide fragment comprises a first peptide fragment, a second peptide fragment, a third peptide fragment, a fourth peptide fragment and a fifth peptide fragment linked in sequence; the first peptide fragment comprises a peptide fragment from AAV1, the second peptide fragment comprises a peptide fragment from AAV7, the third peptide fragment comprises a peptide fragment from AAV2, the fourth peptide fragment is from a peptide fragment comprising AAV1, and the fifth peptide fragment is from peptide fragment comprising AAV6. Preferably, the first peptide fragment comprises an amino acid fragment from position 1 to position 262 of SEQ ID No: 2 (SEQ ID NO: 3), the second peptide fragment comprises an amino acid fragment from position 263 to position 325 of SEQ ID No: 2 (SEQ ID NO: 4), the third peptide fragment comprises an amino acid fragment from position 326 to position 417 of SEQ ID No: 2 (SEQ ID NO: 5), the fourth peptide fragment comprises an amino acid fragment from position 418 to position 583 of SEQ ID No: 2 (SEQ ID NO: 6), the fifth peptide fragment comprises an amino acid fragment from position 584 to position 736 of SEQ ID No: 2 (SEQ ID NO: 7), and the fusion adeno-associated virus capsid protein comprises a S430I mutation.

**TABLE 1. Sequence Information**

| SEQ ID No | Sequence information | Notes |
|---|---|---|
| 1 | atggctgccgatggttatcttccagattggctcgaggacaacctctctgagggcattcgcgagtggtgggacttgaaac ctggagccccgaagcccaaagccaaccagcaaaagcaggacgacggccggggtctggtgcttcctggctacaagta cctcggacccttcaacggactcgacaagggggagcccgtcaacgcggcggacgcagcggccctcgagcacgacaag gcctacgaccagcagctcaaagcgggtgacaatccgtacctgcggtataaccacgccgacgccgagtttcaggagcg tctgcaagaagatacgtcttttgggggcaacctcgggcgagcagtcttccaggccaagaagcgggttctcgaacctct cggtctggttgaggaaggcgctaagacggctcctggaaagaaacgtccggtagagcagtcgccacaagagccagac tcctcctcgggcatcggcaagacaggccagcagcccgctaaaaagagactcaattttggtcagactggcgactcaga gtcagtccccgatccacaacctctcggagaacctccagcaacccccgctgctgtgggacctactacaatggcttcagg cggtggcgcaccaatggcagacaataacgaaggcgccgacggagtgggtaatgcctcaggaaattggcattgcgat tccacatggctgggcgacagagtcatcaccaccagcacccgcacctgggccctgcccacctacaacaatcacctctac aagcaaatctccagtgaaactgcaggtagtaccaacgacaacacctacttcggctacagcaccccctgggggtatttt gactttaacagattccactgccacttctcaccacgtgactggcagcgactcatcaacaacaactggggattccggccc aagaagctgcggttcaagctcttcaacatccaggtcaaggaggtcacgcagaatgacggtacgacgacgattgcca ataaccttaccagcacggttcaggtgtttactgactcggagtaccagctcccgtacgtcctcggctcggcgcatcaagg atgcctcccgccgttcccagcagacgtcttcatggtgccacagtatggatacctcaccctgaacaacgggagtcaggc agtaggacgctcttcattttactgcctggaatatttcccatcgcagatgctgagaacgggcaataactttaccttcagct acacctttgaggaagtgcctttccacagcagctacgcgcacagccagatcctggaccggctgatgaatcctctcatcg accaatacctgtattacctgaacagaactcaaaatcagtccggaagtgcccaaaacaaggacttgctgtttagccgtg | nucleotide sequence |
| | ggtctccagctggcatgtctgttcagcccaaaaactggctacctggaccctgttatcggcagcagcgcgtttctaaaac aaaaacagacaacaacaacagcaattttacctggactggtgcttcaaaatataacctcaatgggcgtgaatccatca tcaaccctggcactgctatggcctcacacaaagacgacgaagacaagttctttcccatgagcggtgtcatgatttttgg aaaagagagcgccggagcttcaaacactgcattggacaatgtcatgatcacagacgaagaggaaatcaaagccact aaccccgtggccaccgaaagatttgggactgtggcagtcaatctccagagcagcagcacagaccctgcgaccggag atgtgcatgttatgggagccttacctggaatggtgtggcaagacagagacgtatacctgcagggacccatttgggcca agattcctcacaccgatggacactttcatccttctccactgatgggaggttttgggctcaaacacccgcctcctcagatc ctcatcaaaaacacgcctgttcctgcgaatcctccggcggagttttcagctacaaagtttgcttcattcatcacccaata ctccacaggacaagtgagtgtggaaattgaatgggagctgcagaaagaaaacagcaagcgctggaatcccgaagt gcagtacacatccaattatgcaaaatctgccaacgttgattttactgtggacaacaatggactttatactgagcctcgcc ccattggcacccgttaccttacccgtcccctgtaa | |
| 2 | MAADGYLPDWLEDNLSEGIREWWDLKPGAPKPKANQQKQDDGRGLVLPGYKYLGPFNGL DKGEPVNAADAAALEHDKAYDQQLKAGDNPYLRYNHADAEFQERLQEDTSFGGNLGRAVF QAKKRVLEPLGLVEEGAKTAPGKKRPVEQSPQEPDSSSGIGKTGQQPAKKRLNFGQTGDSES VPDPQPLGEPPATPAAVGPTTMASGGGAPMADNNEGADGVGNASGNWHCDSTWLGDR VITTSTRTWALPTYNNHLYKQISSETAGSTNDNTYFGYSTPWGYFDFNRFHCHFSPRDWQRLI NNNWGFRPKKLRFKLFNIQVKEVTQNDGTTTIANNLTSTVQVFTDSEYQLPYVLGSAHQGCL PPFPADVFMVPQYGYLTLNNGSQAVGRSSFYCLEYFPSQMLRTGNNFTFSYTFEEVPFHSSYA HSQILDRLMNPLIDQYLYYLNRTQNQSGSAQNKDLLFSRGSPAGMSVQPKNWLPGPCYRQ QRVSKTKTDNNNSNFTWTGASKYNLNGRESIINPGTAMASHKDDEDKFFPMSGVMIFGKES AGASNTALDNVMITDEEEIKATNPVATERFGTVAVNLQSSSTDPATGDVHVMGALPGMVW QDRDVYLQGPIWAKIPHTDGHFHPSPLMGGFGLKHPPPQILIKNTPVPANPPAEFSATKFASF ITQYSTGQVSVEIEWELQKENSKRWNPEVQYTSNYAKSANVDFTVDNNGLYTEPRPIGTRYLT RPL | amino acid sequence |
| 3 | MAADGYLPDWLEDNLSEGIREWWDLKPGAPKPKANQQKQDDGRGLVLPGYKYLGPFNGL DKGEPVNAADAAALEHDKAYDQQLKAGDNPYLRYNHADAEFQERLQEDTSFGGNLGRAVF QAKKRVLEPLGLVEEGAKTAPGKKRPVEQSPQEPDSSSGIGKTGQQPAKKRLNFGQTGDSES VPDPQPLGEPPATPAAVGPTTMASGGGAPMADNNEGADGVGNASGNWHCDSTWLGDR VITTSTRTWALPTYNNHLYKQISS | amino acid sequence |
| 4 | ETAGSTNDNTYFGYSTPWGYFDFNRFHCHFSPRDWQRLINNNWGFRPKKLRFKLFNIQVKE VT | amino acid sequence |
| 5 | QNDGTTTIANNLTSTVQVFTDSEYQLPYVLGSAHQGCLPPFPADVFMVPQYGYLTLNNGSQ AVGRSSFYCLEYFPSQMLRTGNNFTFSYTFE | amino acid sequence |
| 6 | EVPFHSSYAHSQILDRLMNPLIDQYLYYLNRTQNQSGSAQNKDLLFSRGSPAGMSVQPKNW LPGPCYRQQRVSKTKTDNNNSNFTWTGASKYNLNGRESIINPGTAMASHKDDEDKFFPMSG VMIFGKESAGASNTALDNVMITDEEEIKATNPVATERFGTVAVN | amino acid sequence |
| 7 | LQSSSTDPATGDVHVMGALPGMVWQDRDVYLQGPIWAKIPHTDGHFHPSPLMGGFGLKH PPPQILIKNTPVPANPPAEFSATKFASFITQYSTGQVSVEIEWELQKENSKRWNPEVQYTSNYA KSANVDFTVDNNGLYTEPRPIGTRYLTRPL | amino acid sequence |
| 8 | gtcaggcaacgtggcgtggtgtg | nucleotide sequence |
| 9 | ggcgatgagttccgccgtggc | nucleotide sequence |

In the fusion adeno-associated virus capsid protein of the present invention, the peptide fragments are directly fused to each other.

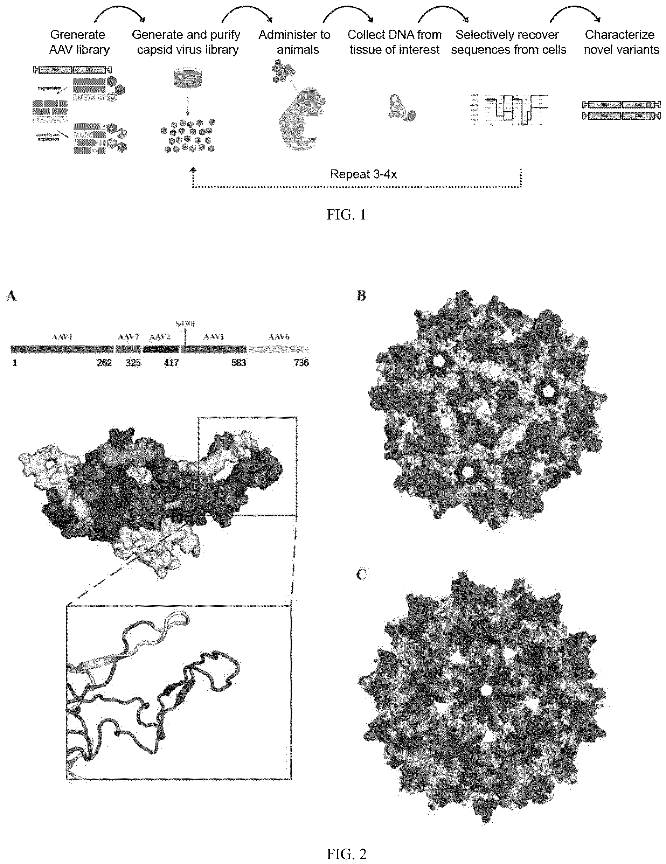

In the fusion adeno-associated virus capsid protein of the present invention, the peptide fragments of the serotypes AAV1 and AAV6 are assembled into protrusions of three-fold symmetry axis of the capsid protein; the peptide fragments of the serotypes AAV2, AAV6 and AAV7 are assembled into channels of five-fold symmetry axis of the capsid protein; the peptide fragment of the serotype AAV6 forms depressions at the two-fold symmetry axis of the capsid protein, and a computer simulation structure diagram thereof is shown in FIG. 2.

Under the electron microscope, the nucleocapsid of adeno-associated virus is generally suborbicular. The suborbicular capsid is actually a closed icosahedral symmetrical hollow capsid composed of a plurality of protein capsomere, in which the genomic nucleic acid is wrapped. One icosahedral symmetric structure comprises three rotational symmetry modes: 3-fold, 2-fold, and 5-fold symmetry. That is, the symmetrical three-dimensional structure has a 3-fold symmetry axis passing through the center points of two opposite sides of the virus particle, around which the capsomere is rotated for three times to be reset to form a triangular surface; a 2-fold symmetry axis (edge), around which the capsomere is rotated for two times to be reset to form two intersecting triangular surfaces; and a 5-fold symmetry axis passing through two opposite vertexes, around which the capsomere is rotated for five times to be reset to form a pentamel. Therefore, the icosahedral symmetrical capsid consists of 20 equilateral triangular surfaces, wherein every 2 triangular surfaces intersect to form edges, for a total of 30 edges; every 5 triangular surfaces are connected to form 12 vertices.

In some preferred embodiments, the capsid protein comprises:
an amino acid sequence as shown in SEQ ID No: 2;
a polypeptide fragment with 90% or more sequence identity to SEQ ID No: 2 and having the function of the amino acid sequence shown in SEQ ID No: 2.

Specifically, the polypeptide fragment in b) specifically refers to a polypeptide fragment obtained by substituting, deleting, or adding one or more (specifically, 1-50, 1-30, 1-20, 1-10, 1-5, 1-3, one, two, or three) amino acids to the amino acid sequence shown in SEQ ID No: 2, or adding one or more (specifically, 1-50, 1-30, 1-20, 1-10, 1-5, 1-3, one, two, or three) amino acids to the N-terminus and/or C-terminus to the amino acid sequence shown in SEQ ID No: 2, and having the function of the amino acid sequence shown in SEQ ID No: 2, the amino acid sequence in b) may have 90%, 91%, 92%, 93%, 94%, 95%, 96%, 97%, 98%, or 99% or more sequence identity to SEQ ID No: 2.

In some preferred embodiments, the nucleic acid sequence of the gene encoding the capsid protein comprises a nucleotide sequence shown in SEQ ID No: 1, preferably, the nucleic acid sequence of the gene encoding the capsid protein is shown in SEQ ID No: 1. In some preferred embodiments, the nucleic acid sequence of the gene encoding the capsid protein comprises a nucleotide sequence having 90%, 91%, 92%, 93%, 94%, 95%, 96%, 97%, 98%, or 99% or more sequence identity to SEQ ID No: 1.

The present invention also provides a nucleic acid encoding the fusion adeno-associated virus capsid protein as described above.

The present invention also provides a construct comprising the above nucleic acid. The construct can generally be obtained by inserting the nucleic acid as described above into a suitable expression vector, and the suitable expression vector can be selected by those skilled in the art.

The present invention further provides a host cell, wherein the host cell comprises the above construct or the genome of the host cell integrates the above exogenous nucleic acid, or the host cell comprises the fusion adeno-associated virus as described in any one of the above.

As representative examples of suitable host cells, mammalian cells (such as CHO or COS), plant cells, human cells (human embryonic kidney cells such as HEK293FT), bacterial cells (such as E. coli, Streptomyces spp, Salmonella typhimurium), fungal cells (such as yeast), insect cells (such as Sf9), and the like, may be exemplified. Those skilled in the art can select a suitable host according to the teachings herein. Preferably, the host cell is an animal cell, and more preferably a human cell. The host cell may be a cultured cell or a primary cell, i.e., cell isolated directly from an organism such as a human. The host cell may be an adherent cell or a suspended cell, i.e., a cell grown in suspension.

The present invention also provides a fusion adeno-associated virus comprising the fused adeno-associated virus capsid protein of any one of the above.

Further, the fusion adeno-associated virus further comprises a heterologous nucleotide sequence encoding the target product, and the heterologous nucleotide sequence encoding the target product can be encapsidated and carried by various capsid proteins. The heterologous nucleotide sequence encoding the target product described above may generally be a construct, which may generally comprise a nucleic acid encoding the target product. The construct may generally be obtained by inserting a nucleic acid encoding a target product into a suitable expression vector, and the suitable expression vector may be selected by those skilled in the art, for example, the expression vector may comprise, but is not limited to, pAAV-CAG, pAAV-TRE, pAAA-EF1a, pAAV-GFAP promoter, pAAA-Lgr5 promoter, pAAA-Sox2 promoter expression vector, and the like. In the present invention, when the fusion adeno-associated virus comprises the heterologous nucleotide sequence encoding the target product, the fusion adeno-associated virus comprises a capsid, the viral vector carries a transgene encoding the gene product, and the transgene is regulated by the regulatory sequence guiding its expression in the host cell; in some preferred embodiments, the amino acid sequence of the capsid protein is shown in SEQ ID NO: 2.

Further, the target product may be a nucleic acid or a protein, the nucleic acid comprises, but is not limited to, small guide RNA (sgRNA), interfering RNA (RNAi) and the like, and the protein encoding gene comprises, but is not limited to, Prestin and Atoh1.

The fusion adeno-associated virus can be used as a vector material to introduce exogenous genes into a cell of a tested individual, and compared with an adeno-associated virus such as AAV-ie, the fusion adeno-associated virus can specifically infect supporting cells of juvenile individuals and specifically infect inner hair cells of adult individuals.

The present invention also provides an engineered host cell obtained by transformation of the fusion adeno-associated virus. The engineered host cell comprises the fusion adeno-associated virus. The host cell may be a eukaryotic cell and/or a prokaryotic cell.

As representative examples of suitable host cells, mammalian cells (such as CHO or COS), plant cells, human cells (human embryonic kidney cells such as HEK293FT), bacterial cells (such as E. coli, Streptomyces spp, Salmonella typhimurium), fungal cells (such as yeast), insect cells (such as Sf9), and the like, may be exemplified. A suitable host may be selected by those skilled in the art according to the teachings herein. Preferably, the host cell is an animal cell, and more preferably a human cell. The host cell may be a cultured cell or a primary cell, i.e., cell isolated directly from an organism such as a human. The host cell may be an adherent cell or a suspended cell, i.e., a cell grown in suspension.

The present invention also provides a fusion adeno-associated virus vector system, the vector system comprises a packaging plasmid, and the packaging plasmid comprises the nucleic acid or the nucleic acid fragment as described above.

Further, the packaging plasmid further comprises a rep gene fragment of adeno-associated virus, wherein the rep gene fragment comprises an intron comprising a transcription termination sequence.

Further, the adeno-associated virus vector system further comprises an expression plasmid comprising a heterologous nucleotide responsible for encoding the target product. Further, the target product may be a nucleic acid or a protein, the nucleic acid comprises, but is not limited to, small guide RNA (sgRNA), interfering RNA (RNAi) and the like, and the protein encoding gene comprises, but is not limited to, Prestin and Atoh1.

Further, the adeno-associated virus vector system further comprises a helper virus plasmid or a helper virus. Further, the adeno-associated virus vector system further comprises a host cell.

The packaging plasmid, the expression plasmid and the helper virus plasmid are transfected into the host cell, and all nucleic acid sequences are integrated into the host cell to produce the fusion adeno-associated virus. In some embodiments, the nucleic acid sequences are all integrated together at a single locus within the cellular genome of the host cell. In some embodiments, the nucleic acid sequences encoding the various genes are present as separate expression cassettes, which prevent any risk of recombination to form a virus capable of replication; the nucleic acid sequences encoding the rep and cap genes are present in the same expression cassette.

The present invention also provides a fusion adeno-associated virus, which is obtained by virus packaging of the fusion adeno-associated virus vector system as described above.

The present invention also provides a pharmaceutical composition comprising the fusion adeno-associated virus as described above and a pharmaceutically acceptable auxiliary material. The fusion adeno-associated virus or pharmaceutical composition provided by the present invention can be adapted to a suitable administration mode, and can be injected into cochlea, eye, muscle, nervous system or blood circulatory system. Those skilled in the art can select an appropriate dosage according to an administration mode.

The auxiliary material may comprise various excipients and diluents, which are not essential active ingredients *per se* and have no excessive toxicity after administration. Auxiliary material should be well known to those skilled in the art. The acceptable carriers are, for example, sterile water or physiological saline, stabilizers, excipients, antioxidants (ascorbic acid, etc.), buffers (phosphoric acid, citric acid, other organic acids, etc.), preservatives, surfactants (PEG, Tween, etc.), chelating agents (EDTA, etc.), adhesives, etc. Moreover, it may also contain other low molecular weight polypeptides; proteins such as serum albumin, gelatin or immunoglobulin; amino acids such as glycine, glutamine, asparagine, arginine and lysine; sugars or carbohydrate such as polysaccharides and monosaccharides; sugar alcohols such as mannitol or sorbitol. When preparing aqueous solutions for injection, such as physiological saline, isotonic solutions containing glucose or other auxiliary drugs, such as D-sorbitol, D-mannose, D-mannitol, sodium chloride, and suitable solubilizers such as alcohols (ethanol, etc.), polyols (propylene glycol, PEG, etc.), nonionic surfactants (Tween 80, HCO-50), etc. can be used in combination.

In the pharmaceutical composition provided by the present invention, the fusion adeno-associated virus AAV-M9 may be a single active ingredient, or can be combined with one or more other active ingredients useful for treating hearing damage or ophthalmic diseases to form a combined preparation. The other active components can be other various drugs which can be used for treating hearing damage or ophthalmic diseases. The amount of the active ingredient in the composition is generally a safe and effective amount, and the safe and effective amount should be adjustable for those skilled in the art, for example, the amount of the active ingredient of the fusion adeno-associated virus AAV-M9 and the pharmaceutical composition is generally dependent on the body weight of the patient, the type of administration, the condition and severity of the disease, for example, the amount of the bifunctional compound as the active ingredient may generally be 1-1000 mg/kg/day, 1-3 mg/kg/day, 3-5 mg/kg/day, 5-10 mg/kg/day, 10-20 mg/kg/day, 20-30 mg/kg/day, 30-40 mg/kg/day, 40-60 mg/kg/day, 60-80 mg/kg/day, 80-100 mg/kg/day, 100-200 mg/kg/day, 200-500 mg/kg/day, or more than 500 mg/kg/day.

The present invention also provides a conjugate comprising the fusion adeno-associated virus AAV-M9 or a linked bioactive polypeptide as described above.

The present invention also provides use of the fusion adeno-associated virus AAV-M9 capsid protein, nucleic acid, construct, fusion adeno-associated virus AAV-M9, host cell, fusion adeno-associated virus vector system, pharmaceutical composition or conjugate in preparation of a medicament for preventing and/or treating diseases; preferably, use in preparation of a medicament for preventing and/or genetically treating diseases; the diseases includes, but are not limited to, one or more of dysaudia diseases, ophthalmic diseases, inflammation, tumors, metabolic diseases, pain, neurodegenerative inflammatory diseases and the like.

The dysaudia disease is selected from hearing loss, deafness and tinnitus.

The ophthalmic diseases includes, but are not limited to, dry AMD, wet AMD, or choroidal neovascularization (CNV); for example, may be selected from age-related macular degeneration (AMD), choroidal neovascularization (CNV), choroidal neovascularization membrane (CNVM), cystoid macular edema (CME), epiretinal membrane (ERM), and macular hole; myopia related choroidal neovascularization, vascular streak, retinal detachment, diabetic retinopathy, diabetic macular edema (DME), atrophic lesion of retinal pigment epithelial cells (RPE), hypertrophic lesions of retinal pigment epithelial cells (RPE), retinal vein occlusion, choroidal retinal vein occlusion, macular edema; corneal angiogenesis due to hypoxia, pterygium conjunctiva, subretinal edema, and intraretinal edema; or one or more of macular edema due to retinal vein occlusion, retinitis pigmentosa, Stargardt disease, glaucoma, inflammatory disease, cataract, refractory anomalies, keratoconus, retinopathy of prematurity, preocular angiogenesis, corneal angiogenesis after keratitis, corneal transplantation, or keratoplasty; such as Leber congenital amaurosis (LCA), pigment retinal degeneration, Stargardt disease caused by RPE cell related gene mutation. In some preferred embodiments, the ophthalmic disease refers to an RPE layer-related disease, that is, gene therapy of the ophthalmic disease can be achieved by infecting the RPE layer with viruses.

The inflammation is selected from the group consisting of skin inflammation, vascular inflammation, allergy, autoimmune diseases, fibrous tissue formation, scleroderma or graft rejection; and the autoimmune diseases are selected from one or more of rheumatoid arthritis, systemic sclerosis, systemic lupus erythematosus, Sjogren's syndrome, polymyositis and the like.

The cancer is selected from lymphoma, hematologic or solid tumor; specifically selected from one or more of adrenocortical carcinoma, bladder urothelial carcinoma, breast cancer, cervical squamous cell carcinoma, endocervical adenocarcinoma, cholangiocarcinoma, colon adenocarcinoma, lymphatic tumor, diffuse large B-cell lymphoma, esophageal cancer, glioblastoma multiforme, head and neck squamous cell carcinoma, renal chromophobe cell carcinoma, renal clear cell carcinoma, renal papillary cell carcinoma, acute myeloid leukemia, low-garde brain glioma, hepatocellular carcinoma, lung adenocarcinoma, lung squamous cell carcinoma, mesocellular cell carcinoma, ovarian cancer, pancreatic cancer, pheochromocytoma and paraganglioma, prostate cancer, rectal cancer, malignant sarcoma, melanoma, gastric cancer, testicular germ cell tumor, thyroid cancer, thymic cancer, endometrial cancer, uterine sarcoma, uveal melanoma, multiple myeloma, acute lymphoid leukemia, chronic lymphoid leukemia, chronic myeloid leukemia, T-cell lymphoma, B-cell lymphoma tumor cells; preferably, the tumor is one or more of colorectal cancer and/or melanocytoma and the like.

The metabolic diseases are selected from diabetes, including type I and type II diabetes, and diseases and conditions related to diabetes; the metabolic diseases include, but are not limited to, one or more of atherosclerosis, cardiovascular diseases, nephropathy, neuropathy, retinopathy, beta-cell dysfunction, dyslipidemia, hyperglycemia, insulin resistance, chronic obstructive pulmonary disease and the like.

In some more preferred embodiments, the gene therapy refers to the treatment of dysaudia disease. The fusion adeno-associated virus or pharmaceutical composition can achieve the treatment of dysaudia disease by delivering the target product to the hair cells and/or supporting cells of an individual.

In the use of the fusion adeno-associated virus AAV-M9, host cell, vector system, pharmaceutical composition or conjugate of the present invention for delivering the target product to a hair cell and/or supporting cell of an individual, the delivery of the target product may be non-diagnostic therapeutic, e.g., *in vitro,* delivery of the target product to an *ex vivo* hair cell and/or supporting cell. The hair cells generally comprise outer hair cells and/or inner hair cells; preferably, the hair cells are inner hair cells of adult individual.

Further, the target product is a nucleic acid or a protein, and the nucleic acid may be small guide RNA (sgRNA), interfering RNA (RNAi), or the like.

In the present invention, the dysaudia disease may result from cochlear damage caused by environmental factors. Therefore, the present invention also provides the use of the above fusion adeno-associated virus in medicament for treating dysaudia diseases caused by environmental factors in individuals.

Further, the dysaudia disease is hair cell and/or supporting cell related disease. In some preferred embodiments, for juvenile individuals, the dysaudia disease is preferably a supporting cell-related disease. In some preferred embodiments, for adult individuals, the dysaudia disease is preferably an inner hair cell related disease. In some preferred embodiments, the dysaudia disease is prevented or treated by inducing inner hair cell regeneration. In another preferred embodiment, the dysaudia disease is prevented or treated by overexpressing Wnt2b and Atoh1 to induce inner hair cell regeneration.

Further, the dysaudia disease is a disease related gene deficiency, environmental damage or aging, for example, it may be a related disease caused by gene mutation, for example, it may be a related disease caused by noise or drug, for example, it may be a related disease caused by aging.

Further, the dysaudia disease may be a related disease of cell damage and the like, specifically, cochlear hair cell damage, supporting cell damage, etc., and more specifically, may be cochlear hair cell damage caused by gene mutation, supporting cell damage caused by gene mutation, cell damage caused by noise, cell damage caused by drugs, or cell damage caused by aging.

Further, the fusion adeno-associated virus is used as a vector for delivering a target product.

The present invention also provides a method for treating a dysaudia disease, comprising administering to a subject in need thereof an effective amount of the fusion adeno-associated virus of the present invention, the host cell, the vector system or the pharmaceutical composition or conjugate of the present invention. Typically, physicians can determine the actual dose that is most suitable for a single patient and will vary depending on the age, weight, and response of a particular individual.

In the present invention, a patient may be administered the fusion adeno-associated virus or host cell or vector system or pharmaceutical composition of the present invention. Those skilled in the art can determine the appropriate mode and dosage of administration.

The delivery of one or more therapeutic genes by the fusion adeno-associated virus described herein can be used alone or in combination with other therapeutic methods or therapeutic components.

The fusion adeno-associated viruses of the present invention are used to infect cells to deliver genes and/or linked (for example, but not limited to, covalently linked) bioactive polypeptides to cells. Accordingly, the invention provides a method of delivering a transgene to a cell by infecting the cell with one or more fusion adeno-associated viruses or conjugates of the invention, wherein the fusion adeno-associated viruses or conjugates comprise one or more transgenes.

The present invention also provides a method of producing a stable cell line that produces a fusion adeno-associated virus vector, comprising:
introducing a fusion adeno-associated viral vector as defined herein into a culture of a mammalian host cell; and selecting within the culture a mammalian host cells having a nucleic acid sequences encoded in the vector integrated into the endogenous chromosome of the mammalian host cell.

The AAV vector producing cells are mammalian cells. In some embodiments, the mammalian cell is selected from HEK293 cells, CHO cells, Jurkat cells, K562 cells, PerC6 cells, HeLa cells or derivatives thereof. In some embodiments, the mammalian host cell is or is derived from a HEK293 cell. In some embodiments, the HEK293 cell is a HEK293T cell.

The genomic sequences of various serotypes of AAV and the sequences of native ITR, Rep proteins and capsid proteins are known in the art. Such sequences can be found in literature or in public databases such as GenBank. The disclosure thereof is incorporated herein by reference for use in AAV nucleic acids and amino acid sequences.

In the compound of the present invention and use thereof, when the fusion adeno-associated virus is used in combination with other therapeutic agents, the active compound is co-administered with other therapeutic agents. "Co-administration" means simultaneous administration in the same formulation or in two different formulations via the same or different routes, or sequential administration via the same or different routes. "Sequential" administration means that there is a time difference in seconds, minutes, hours, or days between the administration of two or more different compounds.

In certain embodiments, the fusion adeno-associated virus of the present invention and methods thereof may be used to prevent hearing damage, and can be administered as a preventive treatment before hearing damage or after a period of time after exposure to an environment prone to hearing damage.

Unless otherwise defined, all professional and scientific terms used herein have the same meaning as familiar to those skilled in the art.

The term "vector" refers to a macromolecule or combination of macromolecules that contains or is bound to a polypeptide and that can be used to mediate the delivery of the polypeptide to a cell. Illustrative vectors comprise, for example, plasmids, viral vectors, liposomes, or other gene delivery vectors.

The term "AAV" is an abbreviation of an adeno-associated virus and may be used to refer to the virus itself or a derivative thereof.

The term "recombinant AAV vector" refers to AAV vectors containing a heterologous polynucleotide sequence, which is typically a sequence of interest for genetically transformation of cells. In general, at least one, typically two AAV inverted terminal repeats (ITRs) is linked to both termini of a heterologous polynucleotide.

The term "AAV virus" or "AAV virus particle" or "AAV vector particle" refers to a virus particle of an AAV vector containing at least one AAV capsid proteins and one encapsulated polynucleotide.

The term "packaging" refers to a series of intracellular processes that result in the assembly and encapsulation of AAV particles.

The terms AAV "rep" and "cap" genes refer to polynucleotide sequences encoding replication and encapsulation proteins of adeno-associated viruses. AAV rep and cap herein refer to AAV "packaging genes".

The term "helper virus" of AAV refers to a virus that enables AAV to be replicated and packaged by mammalian cells. Various such AAV helper viruses are known in the art, comprising adenoviruses, herpes viruses, and pox viruses (e.g., vaccinia).

The term "infectious" virus or viral particle is capable of delivering polynucleotide components into cells with virophilicity of the viral species. The term need not imply that the virus has any replication capability.

The term "production cell" refers to a cell line having an AAV packaging gene (rep and cap genes), a desired helper viral gene, and a DNA genome of a recombinant AAV vector (e.g., a target transgene flanked by two AAV inverted terminal repeats (ITRs)) stably integrated into the host cell genome.

The terms "comprising" "containing" and the like are to be understood to be inclusive, and not exclusive or exhaustive; that is, "including but not limited to".

The term "subject" generally includes human, non-human primates, such as mammal, dog, cat, horse, sheep, pig, cattle and the like, which may benefit from treatment with the formulation, kit or combination.

The term "therapeutically effective amount" generally refers to an amount capable of achieving the effect of treating a disease as listed above after an appropriate period of administration.

The terms "therapeutic" and "prophylactic" are to be understood in their broadest sense. The term "therapeutic" does not necessarily imply that the mammal is treated until fully recovered. Similarly, "prophylactic" does not necessarily mean that the subject will not eventually contract the disease condition. Thus, treatment and prevention include alleviating symptoms of or preventing or reducing the risk of the occurrence of a particular condition. The term "prevention" is to be understood to mean reducing the severity of the onset of a particular disorder. Treatment may also reduce the severity of existing conditions or the frequency of acute onset.

In the present invention, the subject or individual undergoing therapeutic or prophylactic treatment is preferably a mammal, such as but not limited to human, primate, livestock (e.g. sheep, cattle, horse, donkey, pig), pet (e.g. dog, cat), laboratory test animal (e.g. mouse, rabbit, rat, guinea pig, hamster) or captured wild animal (e.g. fox, deer). The subject is preferably a primate. The subject is most preferably a human.

Before further describing the specific embodiments of the present invention, it should be understood that the protection scope of the present invention is not limited to the following specific embodiments; it should also be understood that the terms used in the examples of the present invention are for describing the specific embodiments and are not intended to limit the protection scope of the present invention; and in the specification and claims of the present invention, unless the context clearly indicates otherwise, the singular forms "a", "an" and "the" include the plural forms.

When the embodiments give ranges of values, it should be understood that, unless otherwise indicated by the present invention, the two endpoints of each range of values and any of the values between the two endpoints are optional. Unless otherwise defined, all technical and scientific terms used in the present invention have the same meaning as commonly understood by those skilled in the art. In addition to the specific methods, devices and materials used in the embodiments, according to the knowledge of the prior art by those skilled in the art and the description of the present invention, any methods, devices and materials similar or equivalent to the methods, devices and materials described in the embodiments of the present invention may be used to implement the present invention.

Unless otherwise indicated, the experimental methods, detection methods, and preparation methods disclosed in the present invention all employ conventional molecular biology, biochemistry, chromatin structure and analysis, analytical chemistry, cell culture, recombinant DNA techniques, and conventional techniques in the related art. These techniques have been well described in existing literature.

### EXAMPLES

### Example 1. Obtaining of Novel Adeno-Associated Virus AAV-M9 (also called AAV-WM01)

### a. Construction of DNA Family Shuffling Library

DNA family shuffling was performed with capsid sequences of 13 AAV serotypes (AAV1, AAV2, AAV3, AAV4, AAV5, AAV6, AAV7, AAV8, AAV9, AAV10, AAV1 1, AAV12, AAV13) found in human and non-human primates as parents. A total of 4 µg of the parental capsid sequences were mixed in equal molar ratios for DNA family shuffling, and treated with 0.04 U DNase I at 25°C for 30 seconds to randomly broke the complete parent capsid sequences into fragments of different lengths. DNA fragments with a size of 100 -500 bp were recovered and purified, and 500 ng of DNA fragments were subjected to primerless PCR to extend to a complete chimeric capsid sequence. After sufficient chimeric capsid sequences were obtained by amplification, the chimeric capsid sequences were recombined to a library vector containing a AAV2 Rep gene and a terminal repeat sequence. The recombinant product was transformed into competent cells by electroporation, a portion of bacterial solution was spread on a plate, colonies grown on the plate were counted, and sequencing was carried out to identify the colonies to determine the diversity of the colonies. The remaining bacterial solution was inoculated into 500 mL of medium and cultured overnight at 37 °C, and the plasmid extracted on the next day was the DNA family shuffling library plasmid. The *in vivo* screening procedure of DNA family shuffling library is shown in FIG. 1.

### b. Packaging of DNA Family Shuffling Library Viruses

The obtained DNA family shuffling library plasmid and adenovirus helper plasmid pHelper (the full-length sequence of the plasmid is as shown in SEQ ID NO: 12 of AAV-ie patent document CN110437317A) were co-transformed into HEK-293T cells in appropriate amount. Each 15 cm dish of cells was transfected with only 20 ng DNA family shuffling library plasmids.

Medium was collected after transfection for 72 hours, and cells and medium were collected after additional 48 hours. The cells were lysed with 110 mM citrate buffer (pH4.2) to release AAV, the supernatant containing the virus was neutralized with 1/5 volume of 2M HEPES after centrifugation, then a final concentration of 8% polyethylene glycol 8000 and 500 mM sodium chloride were added, and the virus was precipitated at 4°C overnight. After centrifugation, the precipitate was resuspended in PBS containing 2 mM Mg²⁺, and a final concentration of 100 U/mL benzonase was added and the nucleic acid was digested at 37°C for at least 1 hour. The virus suspension was purified by ultracentrifugation with iodixanol density gradient solution (15%, 25%, 40% and 60%), and then virus titer was determined by qPCR using AAV2 Rep gene-specific primers (WPRE-F: GTCAGGCAACGTGGCGTGGTGTG (SEQ ID NO.8); WPRE-R: GGCGATGAGTTCCGCCGTGGC (SEQ ID NO.9)).

### c. In Vivo Screening

Novel adeno-associated virus that can efficiently infect cochlear cells were screened by *in vivo* injection into mouse cochlea.

Approximately 1E + 10 ~ 11 vg library virus was injected through the oval window into the cochlea of mice born 2-3 days, after 7-10 days of injection, the mice were euthanized, and the cochlea was collected. At this time, the AAVs that could infect cochlear cells in the library virus entered the cell, and the AAVs that failed to infect the cell were cleared by the immune system and the circulatory system. Therefore, the present invention can recover the capsid genes of AAVs infecting cells from the total DNA of tissue cells. The total tissue DNA was extracted by Trizol, and the capsid genes of AAVs were recovered from the total DNA using PCR method to complete an *in vivo* screening of the AAV library. The fragments obtained by PCR were cloned into the library vector to obtain the next round of library plasmid, and the library plasmid was packaged into a virus again, so that the *in vivo* screening process could be repeated, and after 3-5 rounds of screening, the recovered capsid genes of AAVs were subjected to three-generation sequencing. In this way, information of some AAV mutant capsid genes enriched in cochlear cells was obtained, in which some mutant AAVs with high abundance can theoretically efficiently transduce the cochlear cells. The obtained AAV-M9 as shown in SEQ ID NO: 1 is one of the high-abundance mutant.

The sequencing results showed that the AAV-M9 was a chimera of AAV1, 2, 6 and 7 and contained a S430I mutation. A 3D homology model of the AAV-M9 capsid was generated using residues from its VP3 region through amino acid sequence alignment with its parent serotype. Both the inner surface (B) and the outer surface (C) of the capsid show the presence of capsid fragments from the four parent AAVs, indicating that the three-fold symmetry axis protrusion of AAV-M9 is composed of AAV1 and AAV6, AAV2, 6, 7 are assembled into a channel with a five-fold symmetry axis, and AAV6 forms a depression at the two-fold symmetry axis (FIG. 2). The sequence of the coding gene of the AAV-M9 is shown as **SEQ ID No: 1,** and the amino acid sequence is shown as **SEQ ID No: 2.** The computer simulated AAV-M9 structure diagram is shown in FIG. 2.

### Example 2. Biospy Verification of the Novel Adeno-Associated Virus AAV-M9-CMV-EGFP in Auditory System

### 2.1 Construction of Adeno-Associated Virus AAV-M9-CMV-EGFP

The sequence of the encoding gene of the AAV-M9 obtained from the above sequencing result was synthesized by GENEWIZ to obtain a Rep-Cap plasmid of the AAV-M9, i.e., pAAV-M9. The obtained Rep-Cap plasmid pAAV-M9 of AAV-M9, a genome plasmid pAAV-CMV-EGFP expressing a green fluorescent protein EGFP, and a pHelper plasmid were co-transferred into HEK-293T cells in an appropriate amount, AAV was purified by gradient ultrahigh-speed centrifugation of iodixanol, the virus titer of 1E+12-1E+13 GC/mL was an appropriate concentration, and the viruses were placed at -80°C for later use.

### 2.2 Infection Characteristics of Adeno-associated Virus AAV-M9-CMV-EGFP on Neonatal Mice

The same titers of AAV-ie and AAV-M9-CMV-EGFP were injected into the cochleas of C57BL/6J mice born 2-3 days through the oval window. After 10-14 days of fully virus infection and EGFP expression, mice were killed by dislocation of the cervical spine, the cochleas of the injected ears were removed, placed in 4% paraformaldehyde, and fixed at room temperature for 2 hours. They were washed three times with PBS for 5 minutes each time. The cochleas were softened by decalcifying with 0.5 mM EDTA and soaking at room temperature for 2 hours. The basement membranes were dissected with an ophthalmic scalpel under a stereomicroscope. Cochlear samples were prepared by immunofluorescence staining and labeling hair cells with anti-Myo7a antibody. By imaging with a confocal laser scanning microscope, green fluorescent protein was expressed in the nuclei of virus-infected cells. The excitation light wavelengths were 488 nm and 647 nm, respectively. The biopsy results are shown in FIGs. 3a and 3b. FIG. 3a is a patch diagram of hair cell layer of the cochleas injected with AAV-ie and AAV-M9-CMV-EGFP viruses; FIG. 3b is a patch diagram of supporting cell layer of the cochleas injected with AAV-ie and AAV-M9-CMV-EGFP viruses; wherein the marker protein Myo7a of hair cells is shown in purple, fluorescent protein expressed by viral infection cells is shown in green, FIG. 3c shows statistical data of hair cells and Dieters Cells (a supporting cell) with AAV-ie and AAV-M9-CMV-EGFP viruses, and the results show that AAV-M9-CMV-EGFP specifically infects the supporting cells in cochlea of juvenile mice, but has no infection characteristic on hair cells. Dieters Cells are considered to be the supporting cells with the most regenerative potential, and therefore, AAV-M9 has great potential in gene therapy and hair cell regeneration of supporting cells.

### 2.3 Safety of Adeno-associated Virus AAV-M9-CMV-EGFP on Neonatal Mice

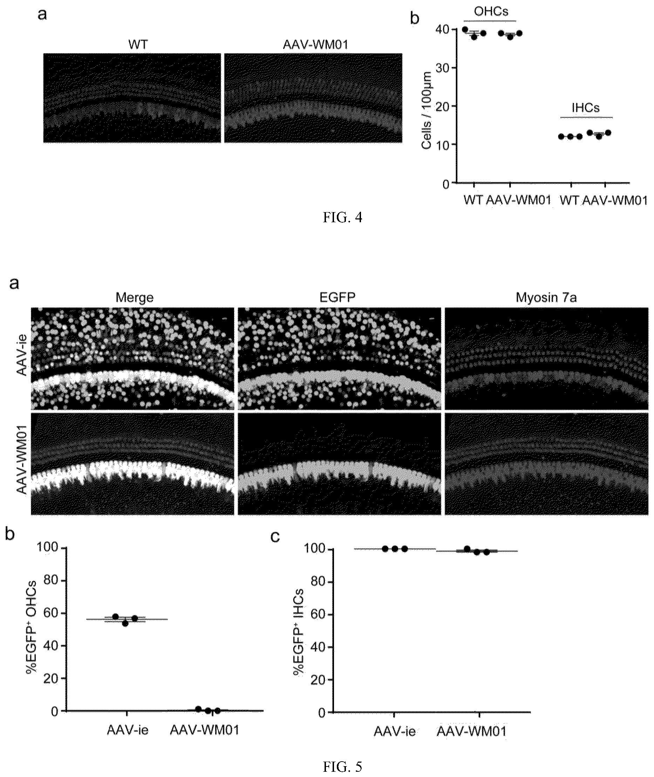

AAV-M9-CMV-EGFP was injected into the cochleas of C57BL/6J mice born 2-3 days through the oval window. After 10-14 days of fully virus infection and expression, mice were killed by dislocation of the cervical spine, the cochleas of the injected ears were removed, and meanwhile, the cochleas of C57BL/6J mice of the same age were taken as a control, the cochleas were placed in 4% paraformaldehyde, and fixed at room temperature for 2 hours. They were washed three times with PBS for 5 minutes each time. The cochleas were softened by decalcifying with 0.5 mM EDTA and soaking at room temperature for 2 hours. The basement membranes were dissected with an ophthalmic scalpel under a stereomicroscope. Cochlear samples were prepared by immunofluorescence staining and labeling hair cells with anti-Myo7a antibody. By imaging with a laser confocal scanning microscope, green fluorescent protein was expressed in the nuclei of virus-infected cells. The biopsy results are shown in FIGs. 4a and 4b. Statistical results show that the number and morphology of cochlear cells of mice injected with the AAV-M9 virus are consistent with the normal mice, indicating that the AAV-M9 virus has no toxicity to neonatal mice.

### 2.4 Infection characteristics of Adeno-associated Virus AAV-M9-CMV-EGFP on Adult Mice

AAV-ie and AAV-M9-CMV-EGFP were injected into the cochleas of C57BL/6J mice born 30 days through the posterior semicircular canal, after 10-14 days of fully virus infection and EGFP expression, the mice were killed by dislocation of the cervical spine, the cochleas of the injected ears were removed, placed in 4% paraformaldehyde, and fixed at room temperature for 2 hours. They were washed three times with PBS for 5 minutes each time. The cochleas were softened by decalcifying with 0.5 mM EDTA and soaking overnight at room temperature. The basement membranes were dissected with an ophthalmic scalpel under a stereomicroscope. Cochlear samples were prepared by immunofluorescence staining and labeling hair cells with anti-Myo7a antibody. By imaging with a laser confocal scanning microscope, green fluorescent protein was expressed in the nuclei of a virus-infected cells. The excitation light wavelengths were 488 nm and 647 nm, respectively. The biopsy result is shown in FIG. 5a. This figure is a patch diagram of the cochleas injected AAV-ie and AAV-M9-CMV-EGFP virus. The labeled protein Myo7a of hair cells was shown in purple, fluorescent protein expressed by virus infected cells is shown in green, and results show that the AAV-M9 specifically infects inner hair cells in cochlea of adult mice, but does not have infection characteristics on outer hair cells, and statistical results show that the infection rate of the AAV-M9 to outer hair cells of adult mice is 0.33% ± 0.333, the infection rate of the AAV-ie to outer hair cells of adult mice is 56.33% ± 1.202, as shown in FIG. 5b; the infection rate of the AAV-M9 to inner hair cells of adult mice is 98.67% ± 0.667, and the infection rate of the AAV-ie to inner hair cells of adult mice is 100% ± 0, as shown in FIG. 5c. The AAV-M9 has high efficiency and specificity in infection of inner hair cells in cochlea of adult mice, and can be used for gene therapy of the inner hair cells.

### 2.5. Safety of Adeno-associated Virus AAV-M9-CMV-EGFP to Adult Mice

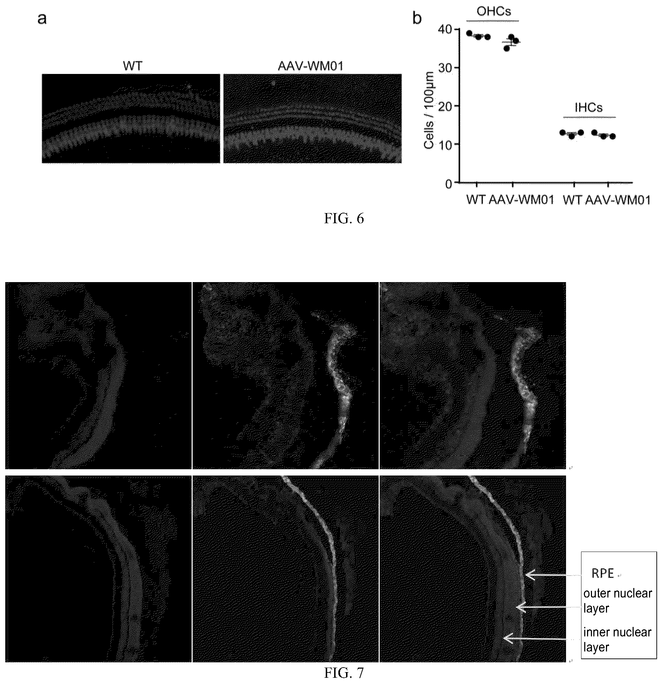

AAV-M9-CMV-EGFP was injected into the cochleas of C57BL/6J mice born 30 days through the oval window. After 10-14 days of fully virus infection and expression, mice were killed by dislocation of the cervical spine, the cochleas of the injected ears were removed, and meanwhile, the cochleas of C57BL/6J mice of the same age were taken as a control, the cochleas were placed in 4% paraformaldehyde, and fixed at room temperature for 2 hours. They were washed three times with PBS for 5 minutes each time. The cochleas were softened by decalcifying with 0.5 mM EDTA and soaking at room temperature for 2 hours. The basement membranes were dissected with an ophthalmic scalpel under a stereomicroscope. Cochlear samples were prepared by immunofluorescence staining and labeling hair cells with anti-Myo7a antibody. By imaging with a confocal laser scanning microscope, green fluorescent protein was expressed in the nuclei of virus-infected cells. The biopsy results are shown in FIGs. 6a and 6b. Statistical results show that the number and morphology of cochlear cells of mice injected with the AAV-M9 virus are consistent with the normal mice, indicating that the AAV-M9 virus has no toxicity to adult mice.

### Example 3. Biospy Validation of Adeno-Associated Virus AAV-M9-CMV-EGFP in Auditory System

Anesthetized adult mouse was placed under a microscope, and 1-2 drops of tropicamide eye drops were dropped on the eyeball to diffuse the pupil. The eyeball was hold up from the optic disc and fixed with lbow tweezers held in the left hand, and a 1 mL syringe or glass needle held in the right hand was used to puncture the cornea to reduce intraocular pressure. The liquid around the eye was wiped off with paper towels. The eyeball was fixed with the tweezers in the left hand, a glass needle with 2 µL of virus held in the right hand was used to slowly inject the adeno-associated virus AAV-M9-CMV-EGFP constructed in 2.1 of Example 2, at the posterior border of the corneosclera at 50° to the iris plane, and the titer was 1.5 E13 gc/mL. After injection, the glass needle was held for 30 s and then slowly pulled out. After the erythromycin ointment was smeared at the wound, the mouse was placed in a cage in a 41 °C water bath for heat preservation, and after the mouse woke up, it was moved to a mouse room for feeding. After 10 days, the eye cup was removed. The mouse was killed by dislocation of the cervical spine, the eyeball was removed and the cornea was punctured by a 1 mL syringe to release the aqueous humor. The eyeball was placed in a small culture dish filled with PBS solution and dissected under a microscope. Pointed tweezers were used to hold the cornea, ophthalmic scissors were poked through the wound in the cornea, the cornea was cut off circumferentially, the lens was removed with tweezers, and the optic nerve was retained by about 2 mm. The eye cup was fixed in 4% PFA for 12 hours at 4°C. The fixed eye cup was dehydrated in 30% sucrose for 12 hours at 4°C. After frozen sectioning, sections with good effect and complete tissues were picked out under a fluorescence microscope for staining. Immunofluorescence results indicate that AAV-M9 can infect the RPE layer. The biospy results are shown in FIG. 7. This figure shows retinal sections injected with AAV-M9-CMV-EGFP virus. The fluorescent protein expressed by virus infection is shown in green, the nuclei are shown in blue, and the results also show that the AAV-M9-CMV-EGFP has higher infection on the retinal RPE layer and can be used for ophthalmic gene therapy.

### Example 4. Characteristics of AAV-M9-CMV-EGFP for Ocular Transfection in Non-Human Primate

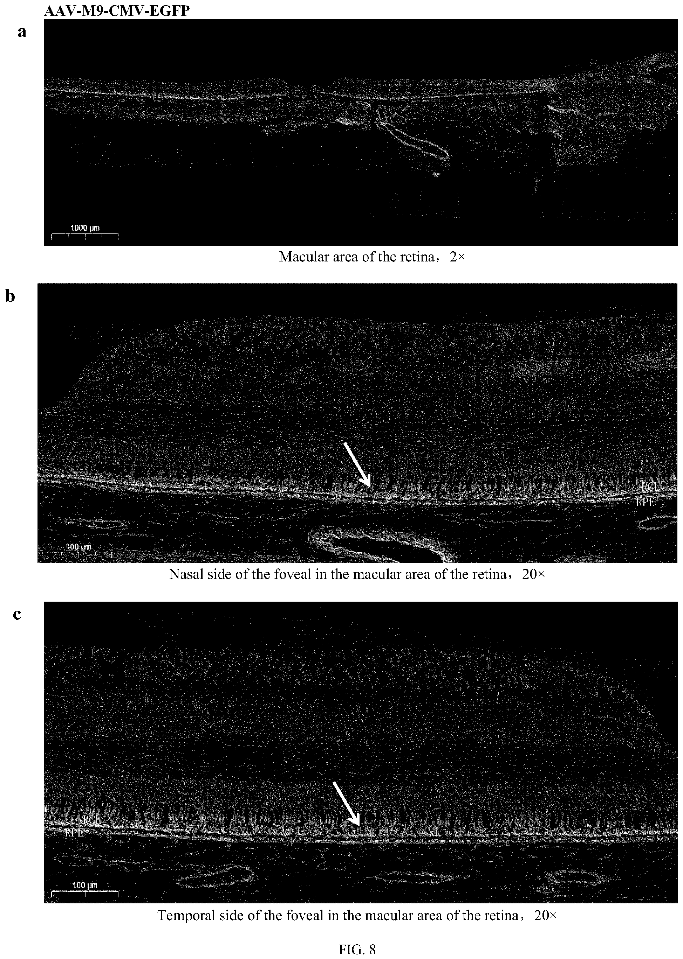

In this experiment, 50 µL of adeno-associated virus AAV-M9-CMV-EGFP was injected into the vitreous of both eyes of adult healthy male rhesus monkeys. The virus titer was 3.89×10¹² vg/mL. On day 63 after virus injection (D63), the animals were euthanized and then the eyeballs were removed, the retina and choroid were subjected to pathological sectioning and staining to analyze the transfection characteristics of the eye tissues. The results are shown in FIG. 8. GFP is mainly expressed at the rod and cone layer (RCL) and pigment epithelial layer (RPE) of the retina, and is also expressed in some vascular endothelial cells in the choroid and/or sclera.

The fusion adeno-associated virus AAV-M9 and the use thereof in prevention and/or treatment of otological diseases or ophthalmic diseases of the present invention effectively overcome various defects in the prior art and have high industrial utilization value.

The above examples are intended to illustrate embodiments of the present disclosure and are not to be construed as a limitation of the present disclosure. Furthermore, various modifications set forth herein, as well as variations of the methods and compositions of the invention, will be apparent to those skilled in the art without departing from the scope and spirit of the invention. While the invention has been described in detail in connection with the various specific preferred embodiments thereof, it should be understood that the invention should not be limited to these specific examples. Indeed, various modifications as described above that would be apparent to those skilled in the art should be inclued within the scope of the invention.

## Claims

1. A fusion adeno-associated virus capsid protein, **characterized in that** the capsid protein comprises a fusion peptide fragment of peptide fragments from serotypes AAV1, AAV2, AAV6 and AAV7, or a variant thereof.

2. The fusion adeno-associated virus capsid protein of claim 1, **characterized in that** the fusion peptide fragment comprises a first peptide fragment, a second peptide fragment, a third peptide fragment, a fourth peptide fragment and a fifth peptide fragment linked in sequence; the first peptide fragment comprises a peptide fragment from AAV1, the second peptide fragment comprises a peptide fragment from AAV7, the third peptide fragment comprises a peptide fragment from AAV2, the fourth peptide fragment is from a peptide fragment comprising AAV1, and the fifth peptide fragment is from a peptide fragment comprising AAV6.

3. The fusion adeno-associated virus capsid protein of claim 1, **characterized in that** the first peptide fragment comprises an amino acid fragment shown in SEQ ID No: 3, the second peptide fragment comprises an amino acid fragment shown in SEQ ID No: 4, the third peptide fragment comprises an amino acid fragment shown in SEQ ID No: 5, the fourth peptide fragment comprises an amino acid fragment shown in SEQ ID No: 6, and the fifth peptide fragment comprises an amino acid fragment shown in SEQ ID No: 7.

4. The fusion adeno-associated virus capsid protein of claim 1, **characterized in** comprising at least any one of the following:
1) the peptide fragments of the serotypes AAV1 and AAV6 are assembled into protrusions of three-fold symmetry axis of the capsid protein;
2) the peptide fragments of the serotypes AAV2, AAV6 and AAV7 are assembled into channels of five-fold symmetry axis of the capsid protein;
3) the peptide fragment of the serotype AAV6 forms depressions at the two-fold symmetry axis of the capsid protein.

5. The fusion adeno-associated virus capsid protein of claim 1, **characterized in that** the capsid protein comprises:
a) an amino acid sequence as shown in SEQ ID No: 2;
b) a polypeptide fragment with 90% or more sequence identity to SEQ ID No: 2 and having the function of the amino acid sequence shown in SEQ ID No: 2.

6. A nucleic acid, **characterized in that** the nucleic acid encodes the fusion adeno-associated virus capsid protein of any one of claims 1 to 5.

7. A construct comprising the nucleic acid of claim 6.

8. A fusion adeno-associated virus, **characterized in that** the capsid structure of the fusion adeno-associated virus comprises the fusion adeno-associated virus capsid protein of any one of claims 1 to 5.

9. The fusion adeno-associated virus of claim 8, **characterized in that** the fusion adeno-associated virus further comprises a heterologous nucleotide sequence encoding a target product; preferably, the target product is a nucleic acid or a protein; more preferably, the nucleic acid comprises a small guide RNA or an interfering RNA.

10. A host cell, **characterized in that** the host cell comprises the construct of claim 7, or the genome of the host cell integrates an exogenous nucleic acid of claim 6, or the host cell comprises the fusion adeno-associated virus capsid protein of any one of claims 1 to 5, or the host cell is transformed with the fusion adeno-associated virus of claim 8 or 9.

11. A fusion adeno-associated virus vector system, **characterized in** comprising a packaging plasmid comprising the nucleic acid of claim 6.

12. The fusion adeno-associated virus vector system of claim 11, **characterized in that** the packaging plasmid further comprises a rep gene fragment of adeno-associated virus.

13. The fusion adeno-associated virus vector system of claim 12, **characterized in that** the adeno-associated virus vector system further comprises an expression plasmid comprising a heterologous nucleotide responsible for encoding a target product; preferably, the target product is a nucleic acid or a protein; more preferably, the nucleic acid comprises a small guide RNA or an interfering RNA.

14. The fusion adeno-associated virus vector system any one of claims 11-13, **characterized in that** the adeno-associated virus vector system further comprises a helper virus plasmid or a helper virus, and the adeno-associated virus vector system further comprises a host cell.

15. A fusion adeno-associated virus obtained by virus packaging of the fusion adeno-associated virus vector system of any one of claims 11 to 14.

16. A pharmaceutical composition or conjugate, **characterized in that** the pharmaceutical composition comprises the fusion adeno-associated virus of any one of claims 8, 9 and 15 and a pharmaceutically acceptable auxiliary material; the conjugate comprises the fusion adeno-associated virus of any one of claims 8, 9 and 15 and a bioactive polypeptide linked thereto.

17. Use of the fusion adeno-associated virus capsid protein of any one of claims 1 to 5, the nucleic acid of claim 6, the construct of claim 7, the fusion adeno-associated virus of any one of claims 8, 9 and 15, the host cell of claim 10, the fusion adeno-associated virus vector system of any one of claims 11 to 14, or the pharmaceutical composition or conjugate of claim 16 in the preparation of a medicament for preventing and/or treating diseases.

18. The use of claim 17, **characterized in that** the disease is selected from one or more of dysaudia disease, ophthalmic disease, inflammation, tumor, metabolic disease, pain and neurodegenerative inflammatory disease.

19. The use of claim 18, **characterized in that** the ophthalmic disease is an RPE layer-related disease, such as dry AMD, wet AMD, or choroidal neovascularization (CNV); such as may be selected from age-related macular degeneration (AMD), choroidal neovascularization (CNV), choroidal neovascularization membrane (CNVM), cystoid macular edema (CME), epiretinal membrane (ERM), and macular hole; myopia related choroidal neovascularization, vascular streak, retinal detachment, diabetic retinopathy, diabetic macular edema (DME), atrophic lesion of retinal pigment epithelial cells (RPE), hypertrophic lesion of retinal pigment epithelial cells (RPE), retinal vein occlusion, choroidal retinal vein occlusion, macular edema; corneal angiogenesis due to hypoxia, pterygium conjunctiva, subretinal edema, and intraretinal edema; macular edema due to retinal vein occlusion, retinitis pigmentosa, Stargardt disease, glaucoma, inflammatory disease, cataract, refractory anomalies, keratoconus, retinopathy of prematurity, preocular angiogenesis, corneal angiogenesis after keratitis, corneal transplantation, or keratoplasty; such as Leber congenital amaurosis (LCA), pigment retinal degeneration, and Stargardt disease caused by RPE cell related gene mutation.

20. The use of claim 17, **characterized in that** the medicament is used for gene therapy of diseases.

21. The use of claim 17, **characterized in** comprising at least one of the following:
1) the dysaudia disease refers to cochlear support cell-related dysaudia disease in a juvenile individual;
2) the dysaudia disease refers to cochlear inner hair cell-related dysaudia disease in an adult individual;
3) the medicament is used to induce regeneration of inner hair cells;
4) the ophthalmic disease is an RPE layer-related disease;
5) the dysaudia disease is caused by cochlear damage;
6) the dysaudia disease is a disease related to cell damage;
7) the dysaudia disease is a related disease caused by gene defects;
8) the dysaudia disease is a related disease caused by environment factors; the environmental factors are selected from noise or otoxicity drugs;
9) the dysaudia disease is a related disease caused by aging.
